Europäisches Patentamt

European Patent Office

Office européen des brevets

(19)

(11) **EP 1 548 426 A1**

(12) # EUROPEAN PATENT APPLICATION
published in accordance with Art. 158(3) EPC

(43) Date of publication:
**29.06.2005 Bulletin 2005/26**

(21) Application number: **03798561.1**

(22) Date of filing: **30.09.2003**

(51) Int Cl.⁷: **G01N 27/22**

(86) International application number:
**PCT/JP2003/012504**

(87) International publication number:
**WO 2004/029607 (08.04.2004 Gazette 2004/15)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **30.09.2002 JP 2002286668**

(71) Applicant: **Mitsui Mining & Smelting Co., Ltd.
Tokyo 141-8584 (JP)**

(72) Inventors:
• **KAWANISHI, Toshiaki,
MITSUI MINING & SMELT. CO.LTD
Ageo-shi, Saitama 362-0021 (JP)**

• **YAMAGISHI, Kiyoshi,
MITSUI MINING & SMELT. C0.LTD
Ageo-shi, Saitama 362-0021 (JP)**
• **TAKAHATA, Takayuki,
MITSUI MINING & SMELT. C0.LTD.
Ageo-shi, Saitama 362-0021 (JP)**

(74) Representative: **Hall, Matthew Benjamin et al
Frank B. Dehn & Co.
179 Queen Victoria Street
London EC4V 4EL (GB)**

(54) **ALCOHOL CONCENTRATION DETECTOR, METHOD OF DETECTING ALCOHOL CONCENTRATION THEREWITH AND PROCESS FOR PRODUCING ALCOHOL CONCENTRATION DETECTION SENSOR**

(57)     It is an object to provide an alcohol concentration detecting apparatus which is small-sized and compact, can be installed everywhere and can have the degree of freedom of a design, has an excellent insulation between electrodes and is not influenced by a moisture, can carry out shielding in order not to be influenced by an electromagnetic wave generated from a body of a car or the like, and furthermore, can execute an accurate measurement for an alcohol concentration.

Furthermore, it is an object to provide an alcohol concentration detecting method using the same apparatus, and an alcohol concentration detecting sensor manufacturing method.

In an alcohol concentration detecting apparatus in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency, the alcohol concentration detecting sensor comprises an alcohol concentration detecting sensor body including a base material resin film, an electrode wiring pattern formed on the base material resin film, and an insulating resin covering a surface of the electrode wiring pattern.

Fig. 2

EP 1 548 426 A1

**Description**

TECHNICAL FIELD

The present invention relates to an alcohol concentration detecting apparatus for detecting an alcohol concentration in a liquid to be inspected, for example, a gasoline and an alcohol concentration detecting method using the same, and an alcohol concentration detecting sensor manufacturing method.

BACKGROUND ART

Conventionally, for example, a so-called high-octane gasoline having an octane value increased such as lead or a benzene based compound, or a gasoline into which an antiknocking agent such as methyl tertiary butyl ether or methyl-t-butyl ether (MTBE) is mixed has been used for a car or the like in order to prevent knocking.

However, there is a possibility that the lead, the benzene based compound or the like might influence an environment. Moreover, it is said that the methyl tertiary butyl ether or the methyl-t-butyl ether (MTBE) is cancer-causing. For this reason, it has been desired to develop the high-octane gasoline and an antiknocking agent in place of the antiknocking agent constituted by the methyl tertiary butyl ether or the methyl-t-butyl ether (MTBE).

Therefore, it has been proposed that alcohol, for example, ethanol is added, as the antiknocking agent, in an amount of approximately 10 to 15% to a gasoline.

However, such ethanol is added so that a torque is reduced. By excessively adding a gasoline corresponding to the amount of addition of the ethanol, therefore, it is necessary to cause the torque to be constant.

For this reason, it has been desired to detect the concentration of alcohol contained in a gasoline.

As a method of detecting the concentration of alcohol, conventionally, an optical alcohol concentration measuring apparatus for detecting the concentration of alcohol by utilizing the refractive index of a light has been disclosed as in Japanese Laid-Open Patent Publication No. Hei 5(1993)-223733 (see paragraphs (0017) to (0030) and Fig. 1) (whichwillbehereinafter referred to as "Patent Document 1").

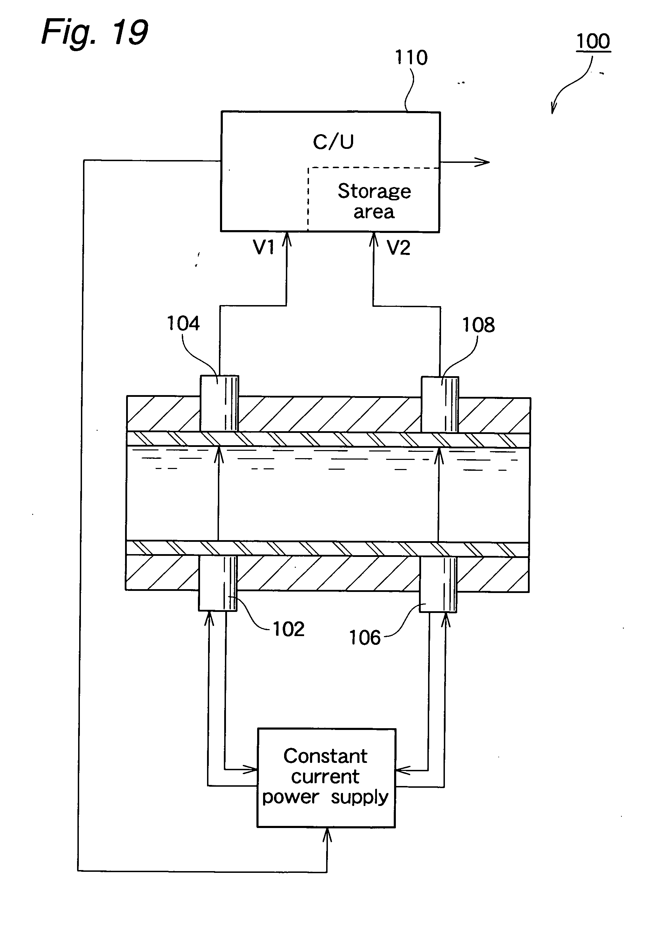

More specifically, in an optical alcohol concentration measuring apparatus 100 in the Patent Document 1, a light which is transmitted from a light emitting portion 102 through a liquid and has a wavelength with such a property that an absorption into alcohol such as ethanol is hard to perform is received by a first light receiving portion 104. Then, a detection signal corresponding to an alcohol concentration in the liquid is output as shown in Fig. 19.

Moreover, a light, which is transmitted from a second light emitting portion 106 through the liquid and has another wavelength with such a property that the absorption into the alcohol is easy, is received by a second light receiving portion 108. Then, a detection signal corresponding to the alcohol concentration in the liquid is output.

Consequently, the detection signal sent from the first light receiving portion 104 is compared with the detection signal sent from the second light receiving portion 108 and the alcohol concentration in the liquid is measured in a measuring portion 110.

As described in "Electrostatic Capacitance Type Alcohol Concentration Sensor" (see Norio Mima, Ikuo Hayashi, Ichiro Hosoya, The Society of Automotive Engineers of Japan, Annual Congress Preliminary Printing Collection 936, 1993-10, pages 257 to 260) (which will be hereinafter referred to as "Non-Patent Document 1"), conventionally, an electrostatic capacitance type alcohol concentration sensor has been proposed.

In the Non-Patent Document 1, there has been proposed a method of measuring the concentration of methanol mixed into a gasoline from an electrostatic capacitance between electrodes at an oscillation frequency by utilizing a difference in a specific inductive capacity between the gasoline and the methanol (the gasoline has a specific inductive capacity of 2 and the methanol has a specific inductive capacity of 33.6), thereby detecting the concentration of the methanol.

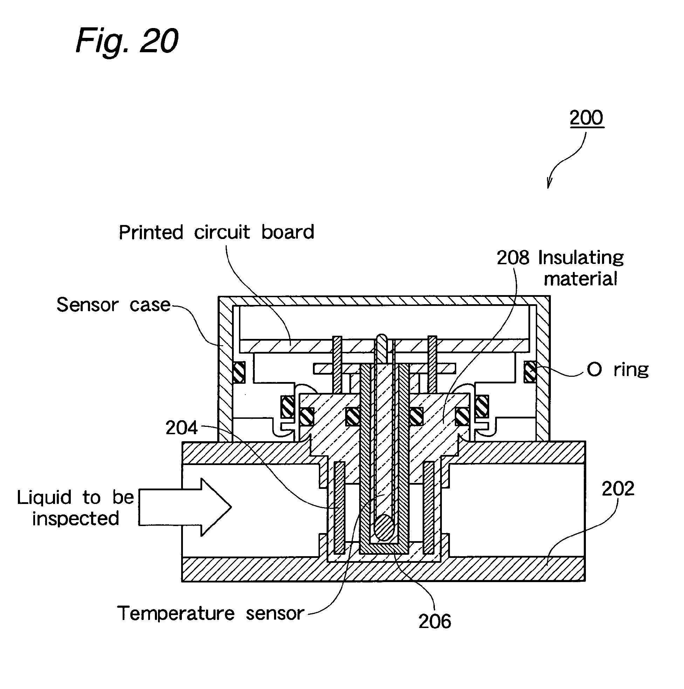

An electrostatic capacitance type alcohol concentration sensor 200 according to the Non-Patent Document 1 has such a structure that an outer electrode 204 and a center electrode 206 are attached through an insulating resin 208 into a housing 202 as shown in Fig. 20.

Since the optical alcohol concentration measuring apparatus according to the Patent Document 1 utilizes a transmitted light, however, it is easily influenced by the composition of a gasoline. For example, in the case in which a liquid to be inspected is not transparent due to an impurity, moreover, a measurement cannot be carried out or an accurate measurement cannot be performed.

In the electrostatic capacitance type alcohol concentration sensor utilizing an electrostatic capacitance according to the Non-Patent Document 1, furthermore, a moisture is apt to enter alcohol and a short circuit is generated between electrodes if the moisture, an electrolyte or the like is present between the electrodes. Accordingly, an insulating treatment for the surface of the electrode is required and a structure thereof is complicated.

In this case, an electrostatic capacitance $C_s$ is expressed in the following equation.

$$C_s = \varepsilon_0(S/D) \left( \varepsilon \, ra \, (\alpha/100) + \varepsilon \, rg(1 - \alpha/100) \right) \qquad \text{Equation 1}$$

Herein, S represents an opposed area of an electrode, D represents a distance between the electrodes, $\varepsilon_0$ represents a specific inductive capacity of a vacuum (8.854E - 12F/m), $\varepsilon$ ra represents a specific inductive capacity of alcohol, $\varepsilon$ rg represents a specific inductive capacity of a gasoline, and $\alpha$ represents an alcohol concentration (%).

As is apparent from the Equation, accordingly, it is preferable to increase the opposed area of the electrode to increase the electrostatic capacitance $C_s$ in order to obtain the excellent results of the measurement. When the opposed area of the electrode is thus increased, however, the size of the electrostatic capacitance type alcohol concentration sensor itself is increased as in the Non-Patent Document 1. For this reason, handling, an application to a car and the like are also restricted in respect of a design.

In the electrostatic capacitance type alcohol concentration sensor according to the Non-Patent Document 1, furthermore, the sensor is to be connected to a body of a gasoline piping in a car or the like, for example. However, a noise such as an electromagnetic wave or the like which is generated from the body influences an alcohol concentration detecting circuit so that an accurate measurement cannot be carried out.

For this reason, an insulating structure is added to the connecting portion of the sensor and the piping or the whole large-sized sensor is to be put in an insulating shield container. Consequently, the apparatus becomes complicated and large-sized.

In consideration of such existing circumstances, it is an object of the present invention to provide an alcohol concentration detecting apparatus for detecting an alcohol concentration in a liquid to be inspected such as a gasoline which is small-sized and compact, can be installed everywhere and can have the degree of freedom of a design, has an excellent insulation between electrodes and is not influenced by a moisture, can carry out shielding in order not to be influenced by an electromagnetic wave generated from a body of a car or the like, and furthermore, can execute an accurate measurement for the alcohol concentration. Furthermore, it is an object to provide an alcohol concentration detecting method using the same apparatus, and an alcohol concentration detecting sensor manufacturing method.

## DISCLOSURE OF THE INVENTION

In order to solve the problems and to attain the object in the conventional art described above, the present invention has been made and provides an alcohol concentration detecting apparatus in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency,

wherein the alcohol concentration detecting sensor comprises an alcohol concentration detecting sensor body including a base material resin film, an electrode wiring pattern formed on the base material resin film, and an insulating resin covering a surface of the electrode wiring pattern.

By such a structure, it is possible to reduce a distance between the electrodes by using the electrode wiring pattern formed on the base material resin film. As is apparent from Equation 2 which will be described below, therefore, an electrostatic capacitance $C_s$ can be increased so that the excellent result of the measurement can be obtained.

In addition, the alcohol concentration detecting sensor is constituted by the base material resin film, the electrode wiring pattern formed on the base material resin film, and the insulating resin covering the surface of the electrode wiring pattern. Therefore, the sensor itself is flexible, thin, very small and compact, and can be installed everywhere so that the degree of freedom of a design can be increased.

Furthermore, the surface of the electrode wiring pattern is covered with the insulating resin. Therefore, an insulation between the electrodes is excellent and is not influenced by a moisture, and shielding can be carried out in order to prevent the influence of an electromagnetic wave generated from the body of a car or the like. Furthermore, an accurate measurement for the alcohol concentration can be executed.

Moreover, the electrode does not directly come in contact with the liquid to be inspected such as a gasoline. Therefore, a defective operation can be prevented from being caused by a deterioration with the passage of time, foreign matters in the gasoline or the like. Thus, it is possible to detect the alcohol concentration accurately and rapidly.

In addition, the alcohol concentration detecting apparatus according to the present invention is characterized in that the alcohol concentration detecting sensor body is stuck onto a substrate.

By such a structure, the alcohol concentration detecting sensor body is stuck onto the substrate. Therefore, it is easy to assemble and attach the alcohol concentration detecting sensor body into the apparatus.

Furthermore, the alcohol concentration detecting apparatus according to the present invention is characterized in that the electrode wiring pattern is obtained by selectively etching a conductive metallic foil laminated on one of surfaces of the base material resin film, thereby forming a wiring pattern taking a predetermines shape.

By such a structure, it is possible to obtain an electrode wiring pattern having a very small distance between the electrodes, for example, within a range of approximately 5 μm to 50 μm by etching. Therefore, the electrostatic capacitance $C_s$ can be increased so that the excellent result of the measurement can be obtained.

In addition, the sensor itself is thin, very small and compact, and can be installed everywhere so that the degree of freedom of a design can be increased.

Moreover, the present invention provides an alcohol concentration detecting apparatus in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency,

wherein the alcohol concentration detecting sensor comprises a substrate, an electrode wiring pattern formed on the substrate, and an insulating coat covering a surface of the electrode wiring pattern.

By such a structure, it is possible to reduce a distance between the electrodes by using the electrode wiring pattern formed on the substrate. As is apparent from the Equation 2 which will be described below, therefore, an electrostatic capacitance $C_s$ can be increased so that the excellent result of the measurement can be obtained.

In addition, the alcohol concentration detecting sensor is constituted by the substrate, the electrode wiring pattern formed on the substrate, and the insulating coat covering the surface of the electrode wiring pattern. Therefore, the sensor itself is thin, very small and compact, and can be installed everywhere so that the degree of freedom of a design can be increased.

Furthermore, the surface of the electrode wiring pattern is covered with the insulating coat. Therefore, an insulation between the electrodes is excellent and is not influenced by a moisture, and shielding can be carried out in order to prevent the influence of an electromagnetic wave generated from the body of a car or the like. Furthermore, an accurate measurement for the alcohol concentration can be executed.

Moreover, the electrode does not directly come in contact with the liquid to be inspected such as a gasoline. Therefore, a defective operation can be prevented from being caused by a deterioration with the passage of time, foreign matters in the gasoline or the like. Thus, it is possible to detect the alcohol concentration accurately and rapidly.

In addition, the substrate is provided. Therefore, it is easy to assemble and attach the alcohol concentration detecting sensor into the apparatus.

Furthermore, the alcohol concentration detecting apparatus according to the present invention is characterized in that the electrode wiring pattern is obtained by selectively etching a conductive metallic thin film formed on one of surfaces of the substrate by sputtering, thereby forming a wiring pattern taking a predetermines shape.

By such a structure, it is possible to obtain an electrode wiring pattern having a thickness of 0.1 to 5 μm by sputtering at a very small distance between the electrodes, for example, within a range of approximately 5 μm to 50 μm by the sputtering. Therefore, the electrostatic capacitance $C_s$ can be increased so that the excellent result of the measurement can be obtained.

In addition, the sensor itself is thin, very small and compact, and can be installed everywhere so that the degree of freedom of a design can be increased.

Moreover, the alcohol concentration detecting apparatus according to the present invention is characterized in that the insulating coat is formed by chemical vapor deposition (CVD).

By such a structure, it is possible to obtain, by the chemical vapor deposition (CVD), a very minute and thin insulating coat which is not influenced by the liquid to be inspected such as a gasoline or alcohol, for example, $SiO_2$, $Al_2O_3$ and the like. Thus, the sensor itself can be thin, very small and compact.

Furthermore, the alcohol concentration detecting apparatus according to the present invention is characterized in that the electrode wiring pattern has such a shape that positive and negative electrodes which are comb-toothed are alternately intricate.

By such a structure, the positive and negative electrodes which are comb-toothed are formed to be alternately intricate. Therefore, the electrodes having a very small distance therebetween can be provided to be compact as a whole.

Accordingly, it is possible to obtain an electrode wiring pattern having a very small distance between the electrodes, for example, within a range of approximately 5 μm to 50 μm by etching and sputtering, respectively. Therefore, the electrostatic capacitance $C_s$ can be increased so that the excellent result of the measurement can be obtained.

In addition, the sensor itself is thinner, much smaller and more compact, and can be installed everywhere so that the degree of freedom of a design can be increased.

Moreover, the present invention provides an alcohol concentration detecting method of detecting an alcohol concentration in a liquid to be inspected by using the alcohol concentration detecting apparatus according to any of the aspects described above,

in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency.

By such a structure, it is possible to reduce a distance between the electrodes by using the electrode wiring pattern formed on the base material resin film or the substrate. As is apparent from the Equation 2 which will be described below, therefore, the electrostatic capacitance $C_s$ can be increased so that the excellent result of the measurement can be obtained.

In addition, the alcohol concentration detecting sensor itself is thin, very small and compact, and can be installed everywhere so that the degree of freedom of a design can be increased.

Furthermore, the surface of the electrode wiring pattern is covered with the insulating resin or the insulating coat. Therefore, an insulation between the electrodes is excellent and is not influenced by a moisture, and shielding can be carried out in order to prevent the influence of an electromagnetic wave generated from the body of a car or the like. Furthermore, an accurate measurement for the alcohol concentration can be executed.

Moreover, the electrode does not directly come in contact with the liquid to be inspected such as a gasoline. Therefore, a defective operation can be prevented from being caused by a deterioration with the passage of time, foreign matters in the gasoline or the like. Thus, it is possible to detect the alcohol concentration accurately and rapidly.

In addition, the alcohol concentration detecting method according to the present invention is characterized in that the liquid to be inspected is a gasoline containing alcohol.

By such a structure, the alcohol concentration in the gasoline can be detected accurately and rapidly, and it is possible to control a torque to be constant by excessively adding a gasoline corresponding to the amount of addition of alcohol, for example, ethanol as an antiknocking agent.

Furthermore, the present invention provides a method of manufacturing an alcohol concentration detecting sensor, comprising:

a conductive metallic foil sticking step of sticking a conductive metallic foil onto one of surfaces of a base material resin film;
a photoresist applying step of applying a photoresist onto a whole upper surface of the conductive metallic foil;
a photoresist exposing step of exposing the photoresist to take a desirable electrode wiring pattern shape by using a photoresist mask;
a photoresist dissolving and removing step of dissolving and removing the exposed photoresist portion with a developing solution;
an etching step of etching and removing a conductive metallic foil portion which is not covered with the photoresist, with an etchant;
a photoresist dissolving and removing step of dissolving and removing the photoresist; and
an insulating resin applying step of applying an insulating resin onto the surface from which the photoresist is removed, thereby obtaining an alcohol concentration detecting sensor body.

By such a structure, it is possible to obtain an electrode wiring pattern having a very small distance between the electrodes, for example, within a range of approximately 5 μm to 50 μm. Consequently, it is possible to easily supply, on a large scale, an alcohol concentration detecting sensor in which the electrostatic capacitance $C_s$ can be increased so that the excellent result of the measurement can be obtained. Furthermore, the sensor itself is thin, very small and compact, and can be installed everywhere so that the degree of freedom of a design can be increased.

In addition, the alcohol concentration detecting sensor manufacturing method according to the present invention comprising a substrate sticking step of sticking the alcohol concentration detecting sensor body obtained at the insulating resin applying step onto a substrate.

By such a structure, the alcohol concentration detecting sensor body is stuck onto the substrate. Consequently, it is possible to easily supply, on a large scale, an alcohol concentration detecting sensor in which the alcohol concentration detecting sensor body can easily be assembled and attached into the apparatus.

Furthermore, the alcohol concentration detecting sensor manufacturing method according to the present invention is characterized in that the base material resin film is a polyimide resin film.

By such a structure, the electrode wiring pattern can-be formed on the flexible, thin and small polyimide resin film. Consequently, it is possible to easily supply, on a large scale, an alcohol concentration detecting sensor in which the sensor itself is thin, very small and compact, and can be installed everywhere and the degree of freedom of a design can be increased.

Moreover, the alcohol concentration detecting sensor manufacturing method according to the present invention is characterized in that the conductive metallic foil is a copper foil.

By such a structure, the electrode wiring pattern can be formed by the copper foil. Consequently, it is possible to easily supply, on a large scale, an alcohol concentration detecting sensor in which a high conductivity can be obtained and an alcohol concentration can be detected very accurately and rapidly.

In addition, the alcohol concentration detecting sensor manufacturing method according to the present invention is characterized in that the insulating resin is constituted by at least one selected from an urethane resin, a polyimide resin and an epoxy type resin.

By using such a resin as the insulating resin, it is possible to easily apply the insulating resin onto the surface of the electrode wiring pattern.

Moreover, the present invention provides a method of manufacturing an alcohol concentration detecting sensor, comprising:

a conductive metallic thin film forming step of forming a conductive metallic thin film on one of surfaces of a substrate by sputtering;

a photoresist applying step of applying a photoresist onto a whole upper surface of the conductive metallic thin film;

a photoresist exposing step of exposing the photoresist to take a desirable electrode wiring pattern shape by using a photoresist mask;

a photoresist dissolving and removing step of dissolving and removing the exposed photoresist portion with a developing solution;

an etching step of dry etching and removing a conductive metallic thin film portion which is not covered with the photoresist;

a photoresist dissolving and removing step of dissolving and removing the photoresist; and

an insulating coat forming step of forming an insulating coat on a surface of the electrode wiring pattern from which the photoresist is removed, by chemical vapor deposition (CVD) .

By such a structure, it is possible to obtain an electrode wiring pattern having a thickness of 0.1 to 5 μm by sputtering at a very small distance between the electrodes, for example, within a range of approximately 5 μm to 50 μm. Therefore, it is possible to easily supply, on a large scale, an alcohol concentration detecting sensor in which the electrostatic capacitance $C_s$ can be increased so that the excellent result of the measurement can be obtained. Furthermore, the sensor itself is thin, very small and compact, and can be installed everywhere so that the degree of freedom of a design can be increased.

Moreover, the alcohol concentration detecting sensor manufacturing method according to the present invention is characterized in that the substrate is constituted by at least one selected from ceramics, glass and a resin substrate.

By such a structure, a conductive metallic thin film constituting the electrode wiring pattern can easily be formed, through sputtering, on the substrate constituted by such a material, and furthermore, the substrate formed by the same material is provided. Consequently, it is possible to easily supply, on a large scale, an alcohol concentration detecting sensor which can easily be assembled and attached into the apparatus.

Furthermore, the alcohol concentration detecting sensor manufacturing method according to the present invention is characterized in that the conductive metallic thin film is constituted by at least one selected from platinum, nickel, copper and titanium.

By such a structure, it is possible to easily form the conductive metallic thin film constituting the electrode wiring pattern on the substrate by sputtering.

In addition, the alcohol concentration detecting sensor manufacturing method according to the present invention is characterized in that the insulating coat is constituted by at least one minute insulating coat selected from $SiO_2$, $Al_2O_3$ and the like.

By such a structure, it is possible to easily form the insulating coat on the surface of the electrode wiring pattern by chemical vapor deposition (CVD).

Moreover, the alcohol concentration detecting sensor manufacturing method according to the present invention is characterized in that the electrode wiring pattern has such a shape that positive and negative electrodes which are comb-toothed are alternately intricate.

By such a structure, the positive and negative electrodes which are comb-toothed are formed to be alternately intricate. Therefore, it is possible to easily supply, on a large scale, an alcohol concentration detecting sensor in which the electrodes having a very small distance therebetween can be provided to be compact as a whole, the sensor itself is thin, very small and compact, and can be installed everywhere so that the degree of freedom of a design can be increased.

BRIEF DESCRIPTION OF THE DRAWINGS

Fig. 1 is a schematic top view showing an example of an alcohol concentration detecting apparatus according to the present invention.

Fig. 2 is a sectional view taken along an A - A line in Fig. 1.

Fig. 3 is a right side view of Fig. 1.

Fig. 4 is a left side view of Fig. 1.

Fig. 5 is a graph showing a relationship between the concentration of alcohol and an electrostatic capacitance.

Fig. 6 is a schematic circuit diagram showing the structure of the alcohol concentration detecting apparatus according to the present invention.

Fig. 7 is a schematic diagram showing a square-wave voltage to be applied by the alcohol concentration detecting apparatus according to the present invention.

Fig. 8 is a graph showing a relationship between an alcohol concentration and an oscillation frequency.

Fig. 9 is a schematic perspective view showing an example of an alcohol concentration detecting sensor in the alcohol concentration detecting apparatus according to the present invention.

Fig. 10 is a schematic top view showing an electrode wiring pattern in Fig. 9.

Fig. 11 is an enlarged view showing a B portion in Fig. 10.

Fig. 12 is a partially enlarged sectional view taken along a C - C line in Fig. 9.

Fig. 13 is a schematic view showing a method of manufacturing the alcohol concentration detecting sensor in the alcohol concentration detecting apparatus according to the present invention.

Fig. 14 is a schematic perspective view showing another example of the alcohol concentration detecting sensor in the alcohol concentration detecting apparatus according to the present invention.

Fig. 15 is a schematic top view showing an electrode wiring pattern in Fig. 14.

Fig. 16 is an enlarged view showing a B portion in Fig. 15.

Fig. 17 is a partially enlarged sectional view taken along a C - C line in Fig. 14.

Fig. 18 is a schematic view showing a method of manufacturing the alcohol concentration detecting sensor in the alcohol concentration detecting apparatus according to the present invention.

Fig. 19 is a schematic view showing a conventional optical alcohol concentration measuring apparatus.

Fig. 20 is a sectional view showing a conventional electrostatic capacitance type alcohol concentration sensor.

BEST MODE FOR CARRYING OUT THE INVENTION

An embodiment(example) of the present invention will be described below in more detail with reference to the drawings.

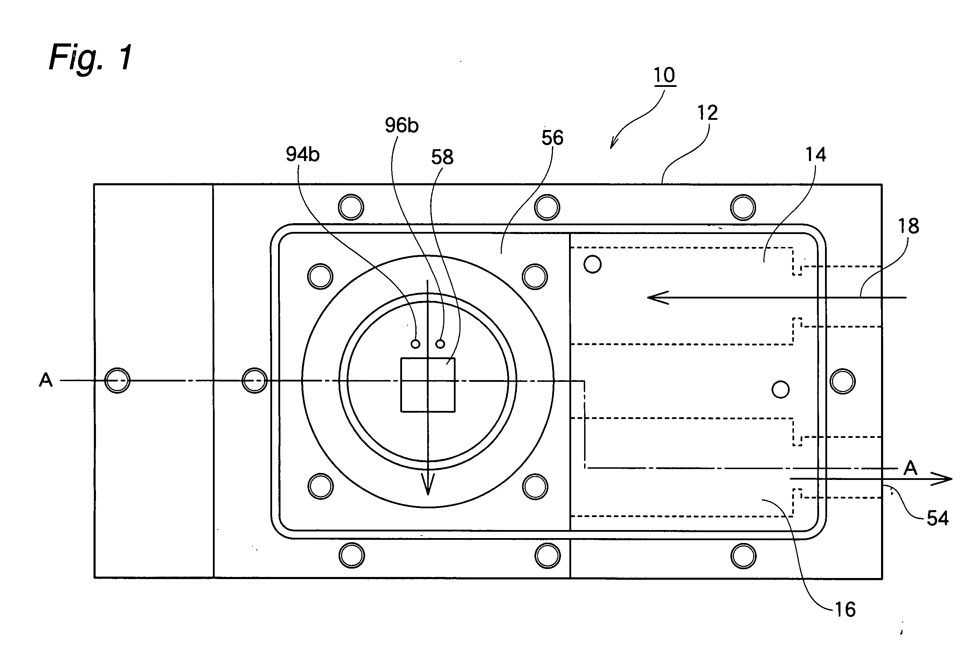

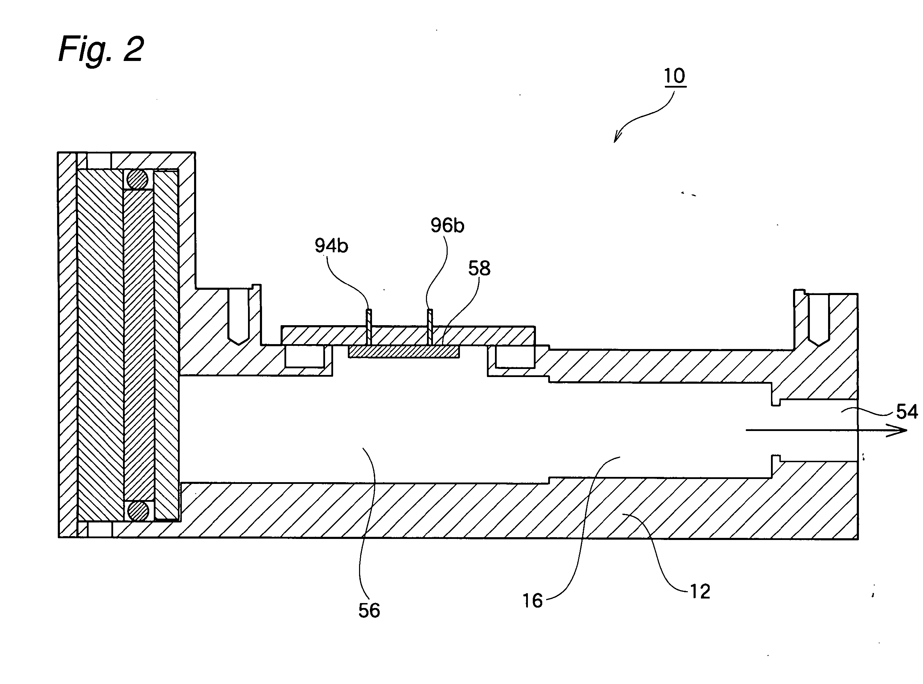

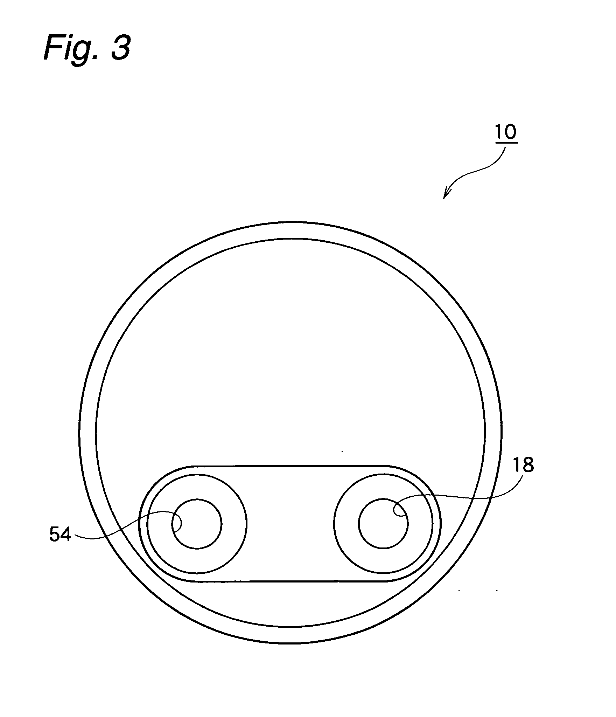

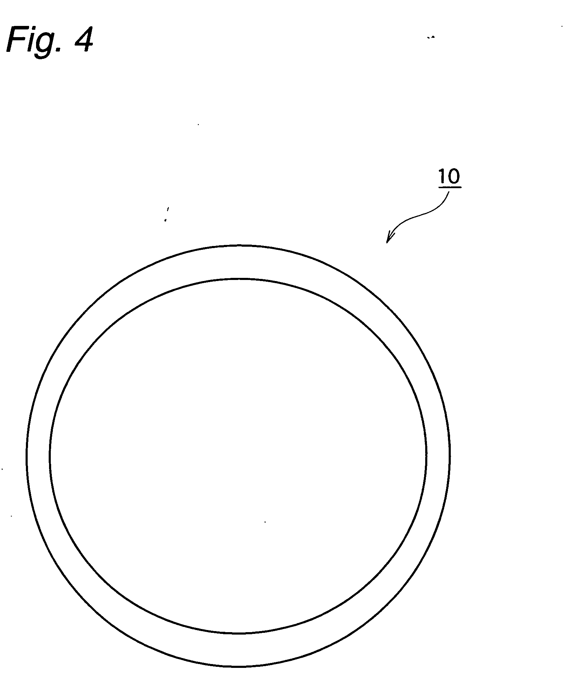

Fig. 1 is a schematic top view showing an example of an alcohol concentration detecting apparatus according to the present invention, Fig. 2 is a sectional view taken along an A - A line in Fig. 1, Fig. 3 is a right side view of Fig. 1, and Fig. 4 is a left side view of Fig. 1.

As shown in Figs. 1 to 4, an alcohol concentration detecting apparatus 10 according to the present invention comprises an alcohol concentration detecting apparatus body 12, and a first passage 14 and a second passage 16 which are formed in the alcohol concentration detecting apparatus body 12.

As shown in an arrow of Fig. 1, a liquid to be inspected which flows from an inspected liquid inlet port 18 into the first passage 14 passes through an alcohol content detecting chamber 56.

On the other hand, in a state in which the liquid to be inspected which flows into the first passage 14 through the inspected liquid inlet port 18 then stays temporarily in the alcoholic content detecting chamber 56, the concentration of the alcohol content is detected by an alcohol concentration detecting sensor 58 if the liquid to be inspected contains alcohol. Then, the same liquid is then discharged from the alcohol content detecting chamber 56 through an inspected liquid discharge port 54 of the second passage 16.

In the alcohol concentration detecting sensor 58, a difference in an electrostatic capacitance is utilized depending on a difference between the specific inductive capacity of alcohol contained in the liquid to be inspected and the specific inductive capacity of the liquid to be inspected based on the following Equation 2.

$$C_s = \varepsilon_0 (S/D)\, (\varepsilon\, ra(\alpha/100) + \varepsilon\, rb(1 - \alpha/100)) \qquad \text{Equation 2}$$

Herein, S represents an opposed area of an electrode, D represents a distance between the electrodes, $\varepsilon_0$ represents a specific inductive capacity of a vacuum (8.854E - 12F/m), $\varepsilon$ ra represents a specific inductive capacity of alcohol, $\varepsilon$ rb represents a specific inductive capacity of a liquid to be inspected, and $\alpha$ represents an alcohol concentration (%).

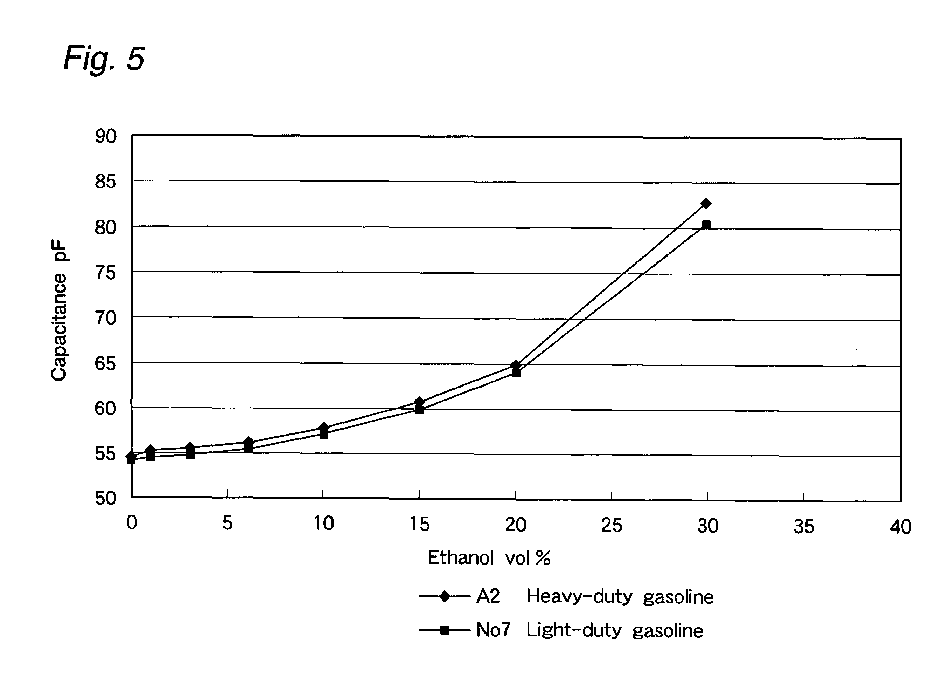

More specifically, as shown in a graph of Fig. 5 which represents a relationship between an alcohol concentration and an electrostatic capacitance, the alcohol concentration and the electrostatic capacitance have a correlation, and the alcohol concentration is detected by utilizing the correlation.

Fig. 5 shows an example in which ethanol is used for the alcohol and a gasoline is used for the liquid to be inspected.

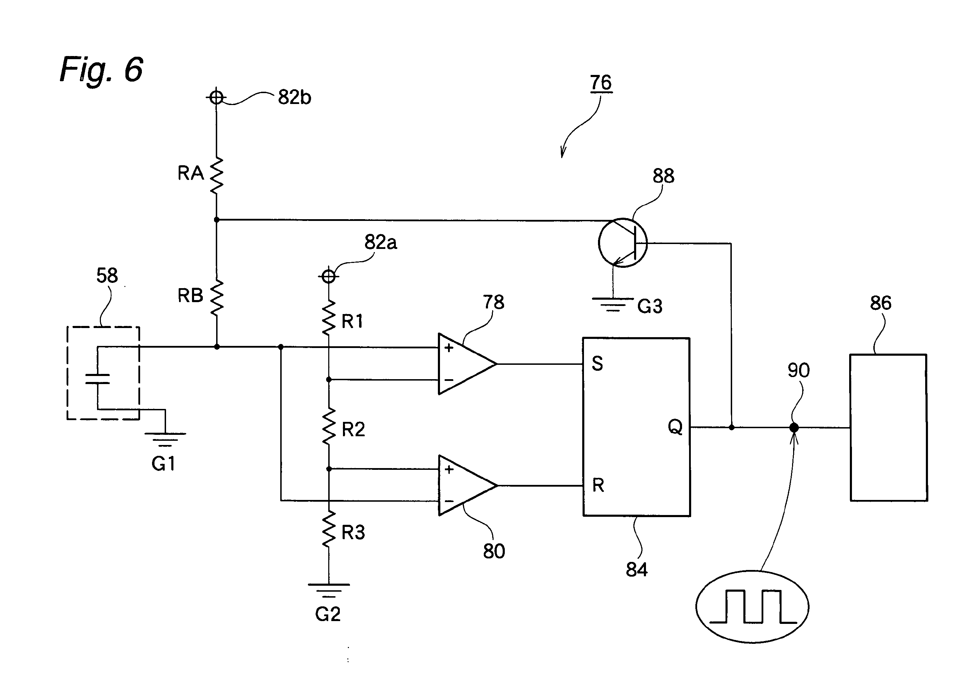

Moreover, the alcohol concentration detecting apparatus 10 according to the present invention which uses the alcohol concentration detecting sensor 58 comprises a detecting control portion 76 having a structure shown in a diagram of Fig. 6 illustrating the schematic structure of a circuit.

As shown in Fig. 6, in the detecting control portion 76, one of the electrodes of the alcohol concentration detecting sensor 58 is grounded G1. In addition, the other electrode of the alcohol concentration detecting sensor 58 branches to be connected to the positive and negative inputs of amplifiers (operational amplifiers) 78 and 80.

Moreover, resistors R1 to R3 are connected to a negative 82a of a power supply 82, and furthermore, the negative input of the amplifier 78 is connected between R1 and R2. Furthermore, the positive input of the amplifier 80 is connected between R2 and R3, and an end of R3 is grounded G2.

The outputs of the amplifiers 78 and 80 are connected to S and R inputs of a flip-flop circuit 84, respectively. The output of the flip-flop circuit 84 is input to the frequency counter of a computer 86.

Furthermore, the wiring of one of the electrodes of the alcohol concentration detecting sensor 58 branches and is connected to a positive 82b of the power supply 82 through resistors RA and RB. A transistor 88 is connected between the resistors RA and RB, and the output of the transistor is connected between the output of the flip-flop circuit 84 and the computer 86. G3 denotes a ground of the transistor 88.

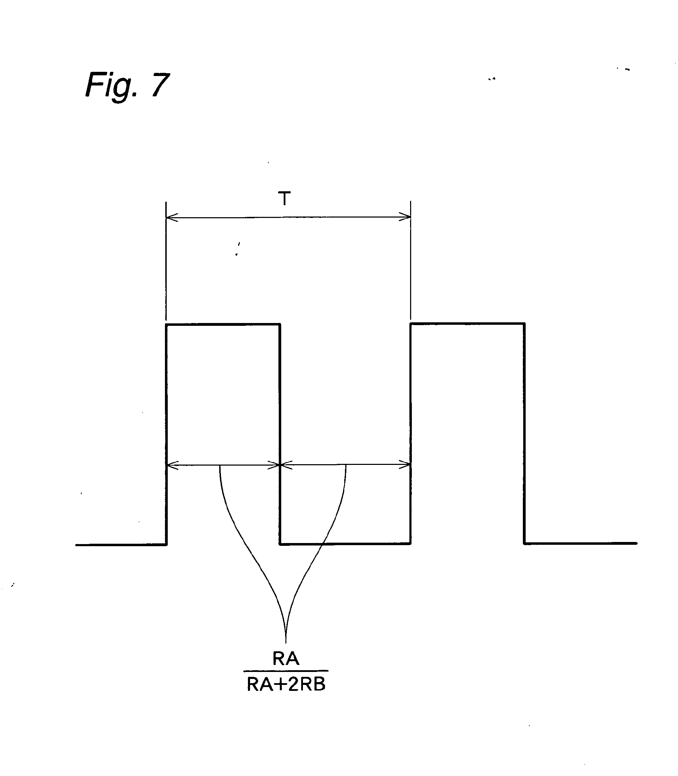

In the detecting control portion 76 having such a structure, a square-wave voltage shown in Figs. 6 and 7 is applied at 90 in Fig. 6.

As is expressed in the following Equation 3, consequently, a relationship between an oscillation frequency f and an electrostatic capacitance $C_s$ is obtained.

$$1/T = f = RA/ (RA + 2RB) \cdot 1/Cs \text{ (Hz)} \qquad \text{Equation 3}$$

In this case, it is possible to determine an amplitude T by properly setting a duty ratio RA/ (RA + 2RB). In the present example, 1.44 was employed for the duty ratio.

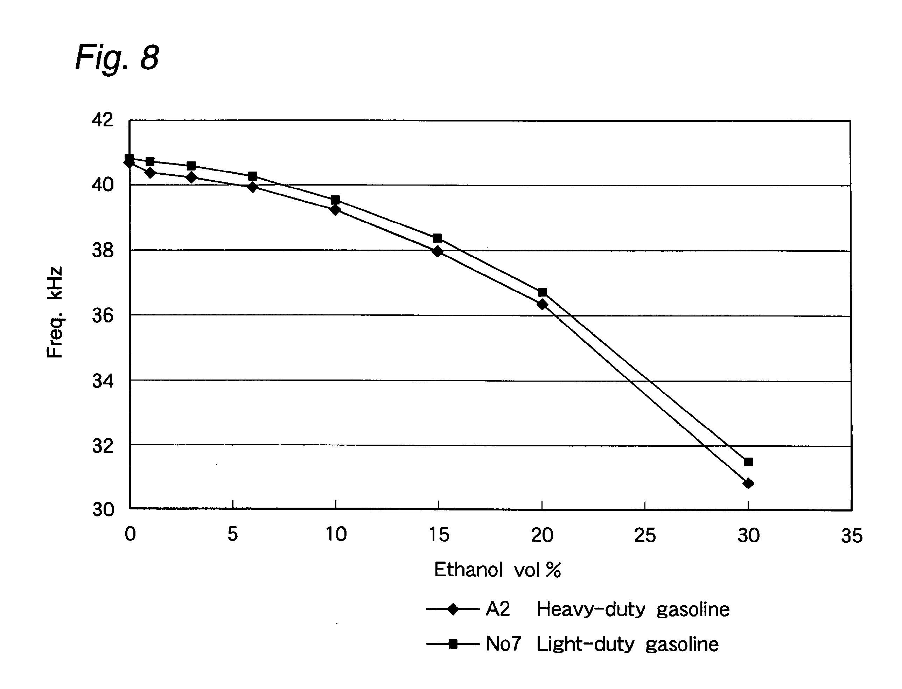

From such a relationship, a correlation is taken based on the graph of Fig. 5. As shown in a graph of Fig. 8 representing a relationship between an alcohol concentration and an oscillation frequency, consequently, it is apparent that the alcohol concentration and the oscillation frequency have a correlation. Thus, it is possible to detect the alcohol concentration.

The data in Figs. 5 and 8 are previously stored in the storage portion of the computer and are compared with data obtained in the detecting control portion 76 so that the alcohol concentration can be detected.

As is apparent from the Equation 2, in order to obtain the excellent result of a measurement, it is preferable that the distance D between electrodes should be reduced to increase the electrostatic capacitance $C_s$.

In the alcohol concentration detecting apparatus 10 according to the present invention, therefore, the alcohol concentration detecting sensor 58 is constituted in the following manner.

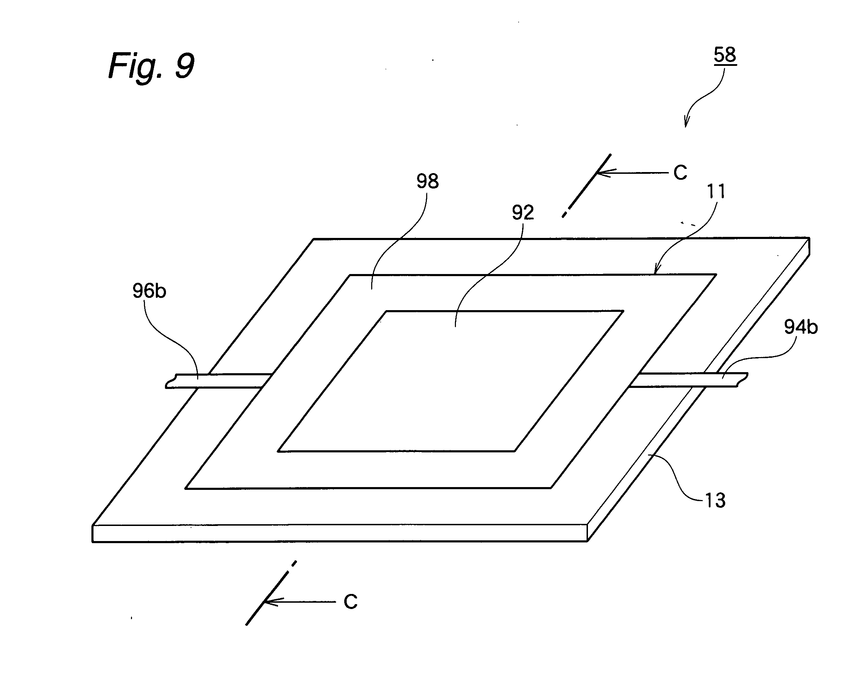

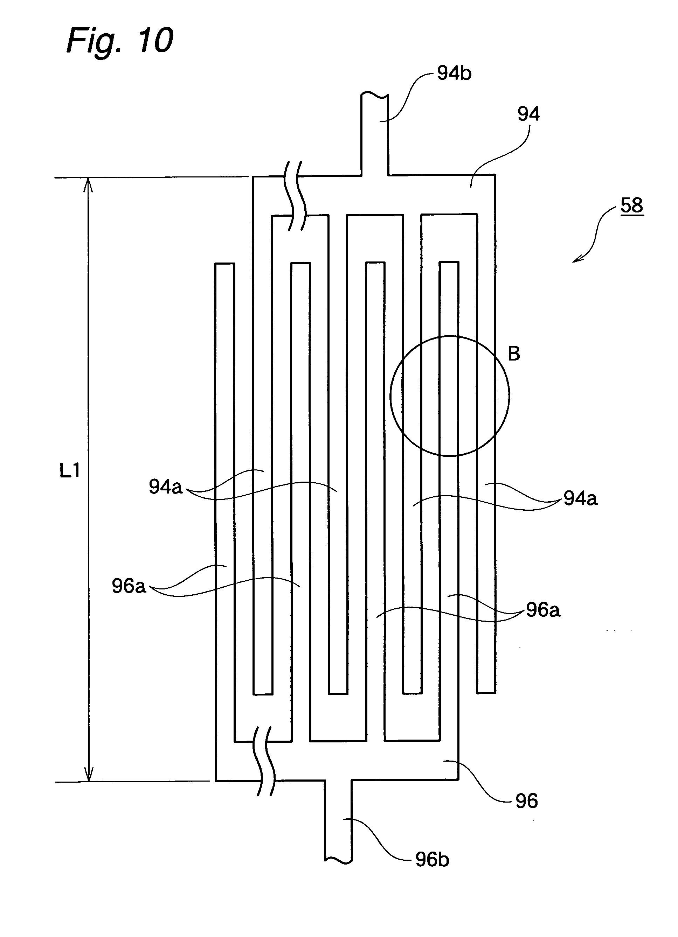

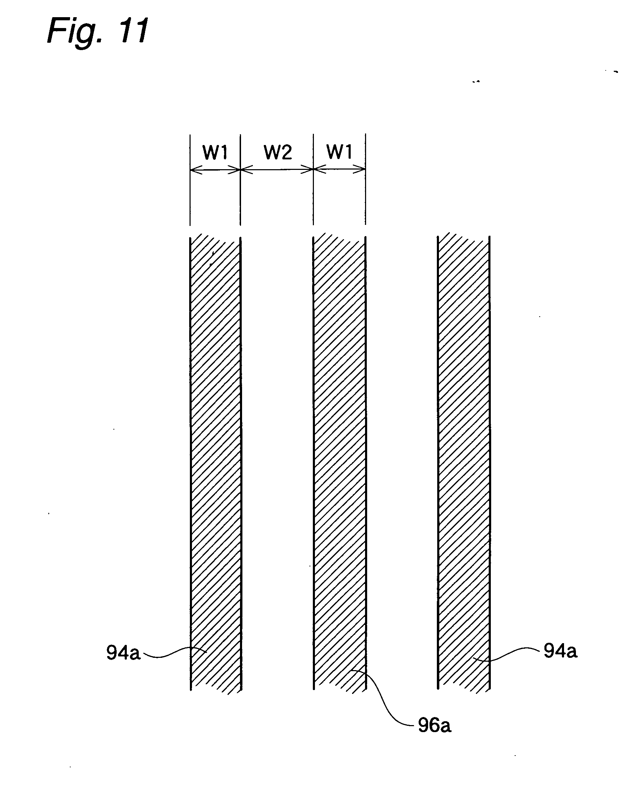

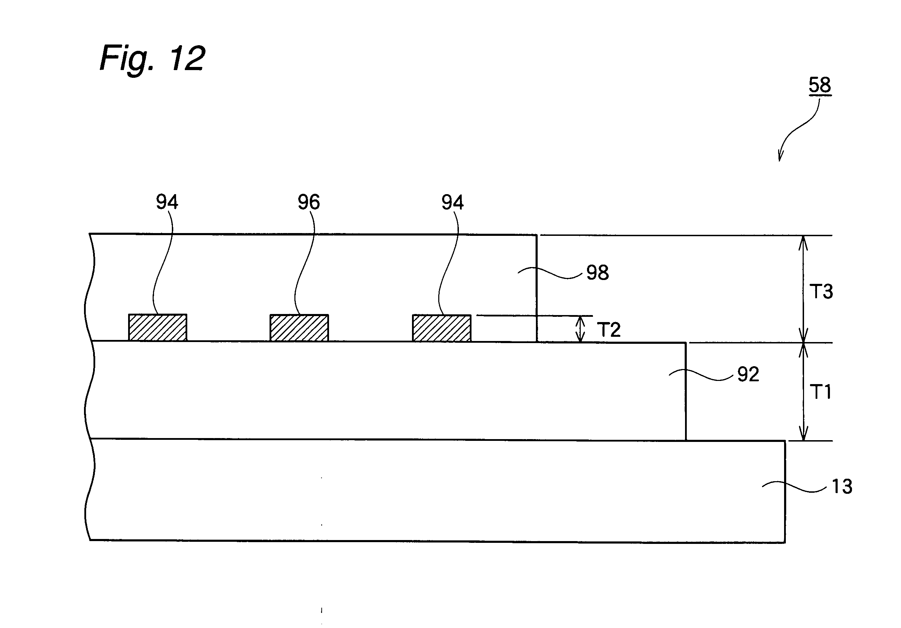

More specifically, Fig. 9 is a schematic perspective view showing an example of the alcohol concentration detecting sensor 58 of the alcohol concentration detecting apparatus according to the present invention, Fig. 10 is a schematic top view showing an electrode wiring pattern in Fig. 9, Fig. 11 is an enlarged view showing a B portion in Fig. 10, and Fig. 12 is a partially enlarged sectional view taken along a C - C line in Fig. 9.

As shown in Figs. 9 to 12, the alcohol concentration detecting sensor 58 includes an alcohol concentration detecting sensor body 11 constituted by a base material resin film 92, electrode wiring patterns 94 and 96 formed on the base material resin film 92, and an insulating resin 98 covering the surfaces of the electrode wiring patterns 94 and 96. The alcohol concentration detecting sensor body 11 is stuck to a substrate 13 with an adhesive which is not shown.

In this case, it is preferable that a polyimide resin film shouldbe used for the basematerial resin film 92 in consideration of a flexibility, a chemical resistance and the like. As shown in Fig. 12, moreover, a thickness T1 is not particularly restricted.

Furthermore, the electrode wiring pattern 94 on a positive side and the electrode wiring pattern 96 on a ground (a negative side) have such shapes that positive electrodes 94a and negative electrodes 96a which are comb-toothed are alternately intricate, respectively. In Fig. 9, 94b and 96b denote a fetch electrode portion, respectively.

By such a structure, a plurality of electrodes having a very small distance therebetween can be provided to be compact as a whole.

In this case, while a length L1 of the electrode is not particularly restricted as shown in Fig. 10, it is desirably set to be 100μm or more in consideration of the electrostatic capacitance of the liquid tobe inspected. In the present example, a length of 10 mm was employed for L1.

While a width W1 of each of the positive electrode 94a and the negative electrode 96a is not particularly restricted as shown in Fig. 11, moreover, in consideration of the electrostatic capacitance, it is preferably set to be 1 to 50 µm, and more preferably, 5 to 15 µm. Furthermore, a width W2 between the positive electrode 94a and the negative electrode 96a is not particularly restricted but is preferably set to be 1 to 50 µm, and more preferably 5 to 15 µm in consideration of the electrostatic capacitance. In the present example, W1/W2 = 30/30 µm was employed.

While the numbers of the positive electrodes 94a and the negative electrodes 96a which are comb-toothed are not particularly restricted, moreover, in consideration of the electrostatic capacitance, they are preferably equal to or larger than 1, and more preferably, are more increased. In the present example, 64 pairs (128 in total) comb-toothed electrodes were used.

While a thickness T2 of each of the electrode wiring patterns 94 and 96 is not particularly restricted as shown in Fig. 12, furthermore, in consideration of the electrostatic capacitance, it is preferably set to be 1 to 50 µm, and more preferably, 5 to 15 µm. In the present example, T2 of 10 µm was employed.

In this case, the electrode wiring patterns 94 and 96 are obtained by selectively etching a conductive metallic foil laminated on one of the surfaces of the base material resin film 92 to form wiring patterns taking predetermined shapes as will be described below.

Although such a conductive metallic foil is not particularly restricted, a copper foil is preferable. Consequently, a high conductivity can be obtained and the concentration of alcohol can be detected very accurately and rapidly.

Furthermore, it is preferable that the insulating resin 98 should be formed by at least one insulating resin selected from an urethane resin, a polyimide resin and an epoxy type resin.

By using such a resin as the insulating resin 98, it is possible to easily apply the insulating resin onto the surfaces of the electrode wiring patterns 94 and 96.

While a thickness T3 of the insulating resin 98 is not particularly restricted as shown in Fig. 12, moreover, in consideration of the fact that the electrostatic capacitance of the insulating resin itself does not influence sensing, it is desirable that the thickness T3 should be smaller with an insulating property and a strength maintained. In the present example, T3 of 18 µm was employed.

While the material of the substrate 13 is not particularly restricted, furthermore, in consideration of a specific inductive capacity, it is possible to employ a glass substrate, a ceramics substrate, a resin substrate or the like. Although the thickness is not particularly restricted, it is preferably set to be 100 to 1000 µm, and more preferably, 250 to 600 µm in consideration of an insulating property, a strength and the like. In the present example, a thickness of 360 µm was employed.

By such a structure, it is possible to reduce the distance between the electrodes by using the electrode wiring patterns 94 and 96 formed on the base material resin film 92. As is apparent from the Equation 2, therefore, the electrostatic capacitance $C_s$ can be increased so that the excellent result of the measurement can be obtained.

In addition, the alcohol concentration detecting sensor 58 is constituted by the base material resin film 92, the electrode wiring patterns 94 and 96 formed on the base material resin film 92, and the insulating resin 98 covering the surfaces of the electrode wiring patterns 94 and 96. For this reason, the sensor itself is flexible, thin, very small and compact, and can be installed everywhere so that the degree of freedom of a design can be enhanced.

Since the surfaces of the electrode wiring patterns 94 and 96 are covered with the insulating resin 98, furthermore, an insulation between the electrodes can be enhanced and is not influenced by a moisture, and shielding can be carried out so as not to be influenced by an electromagnetic wave generated from a body of a car or the like. Furthermore, the alcohol concentration can be measured accurately.

Moreover, the electrode does not directly come in contact with the liquid to be inspected such as a gasoline. Therefore, a defective operation can be prevented from being caused by a deterioration with passage of time, foreign matters in the gasoline or the like. Thus, it is possible to detect the alcohol concentration accurately and rapidly.

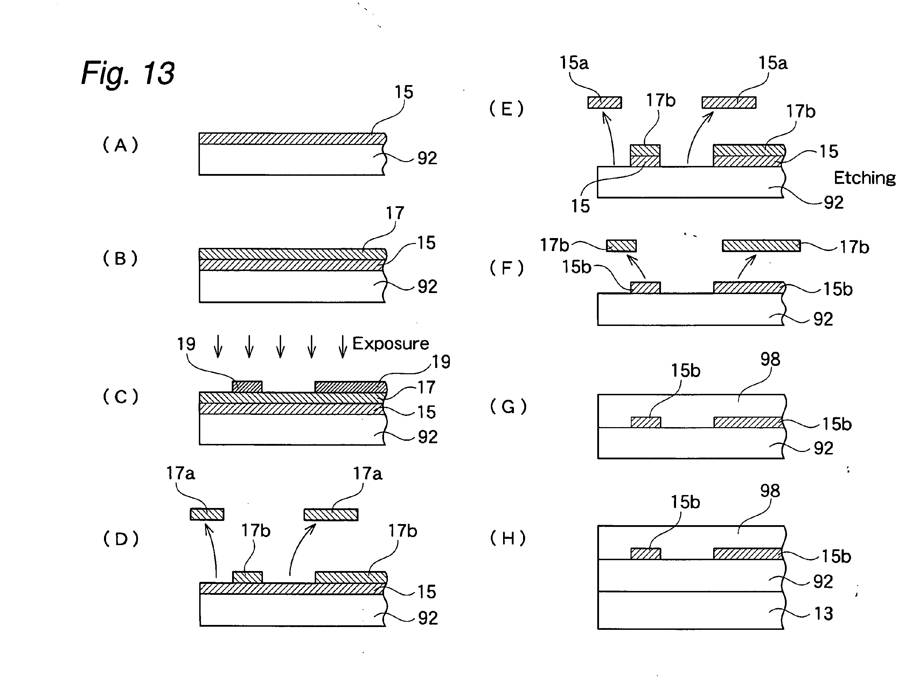

With reference to Fig. 13, description will be given to a method of manufacturing the alcohol concentration detecting sensor of the alcohol concentration detecting apparatus according to the present invention which has such a structure.

As shown in Fig. 13 (A), first of all, a conductive metallic foil 15 is stuck to one of the surfaces of the base material resin film 92 by contact bonding with an adhesive which is not shown (a conductive metallic foil sticking step).

As shown in Fig. 13 (B), then, a photoresist 17 is applied onto the whole upper surface of the conductive metallic foil 15 by using a spin coater (3000 rpm), for example (a photoresist applying step).

As shown in Fig. 13 (C), next, the photoresist 17 is exposed by ultraviolet rays to take a desirable electrode wiring pattern shape by using a photoresist mask 19 taking a shape corresponding to a predetermined wiring pattern, for example (a photoresist exposing step).

As shown in Fig. 13(D), then, a photoresist portion 17a thus exposed is dissolved and removed with a developing solution (a photoresist dissolving and removing step).

As shown in Fig. 13 (E), thereafter, a conductive metallic foil portion 15a which is not covered with the photoresist 17b is subjected to an etching treatment with an etchant such as acid or alkali and is thus removed to obtain a predetermined wiring pattern shape 15b (an etching step).

As shown in Fig. 13 (F), subsequently, the photoresist 17b is dissolved and removed with a dissolving and removing solution such as acetone (a photoresist dissolving and removing step).

As shown in Fig. 13(G), next, the insulating resin 98 is applied onto the surface from which the photoresist is removed by screen printing, for example, and the alcohol concentration detecting sensor body 11 is thus obtained (an insulating resin applying step).

As shown in Fig. 13 (H) and Fig. 12, finally, the alcohol concentration detecting sensor body 11 obtained at the insulating resin applying step is stuck onto the substrate 13 (a substrate sticking step).

According to the method of manufacturing the alcohol concentration detecting sensor 58 of the alcohol concentration detecting apparatus according to the present invention, it is possible to obtain an electrode wiring pattern in which a distance between electrodes is very small, for example, approximately 5μm to 50μm. Therefore, it is possible to easily supply, on a large scale, an alcohol concentration detecting sensor in which the electrostatic capacitance $C_s$ can be increased and the excellent result of the measurement can be obtained. Furthermore, the sensor itself is thin, very small and compact, and can be installed everywhere so that the degree of freedom of a design can be enhanced.

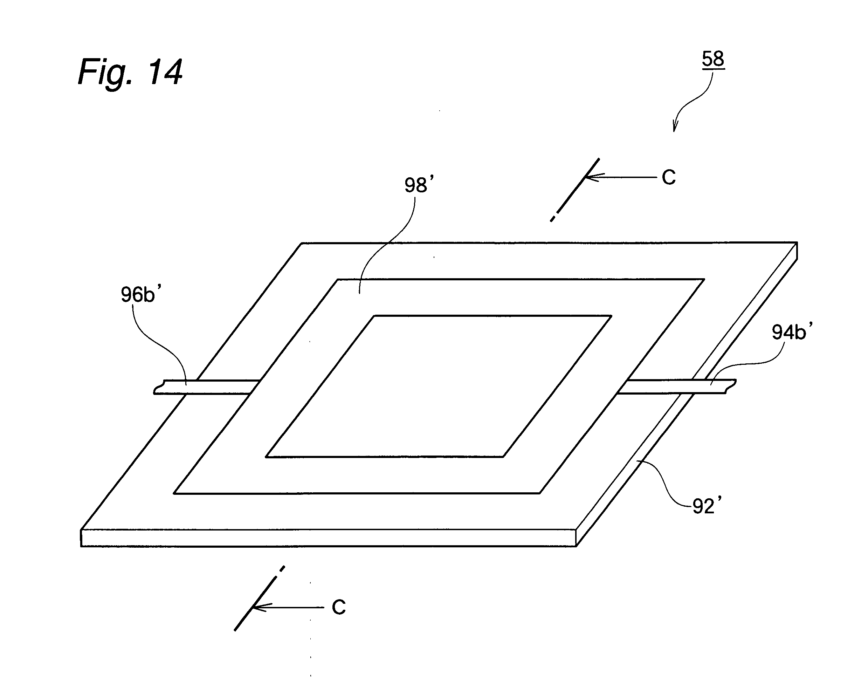

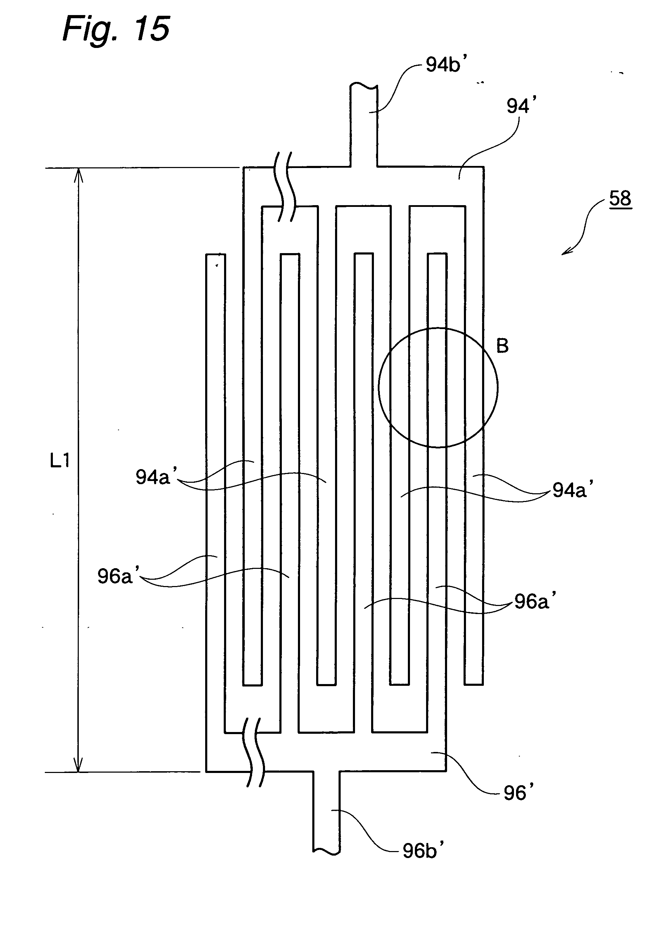

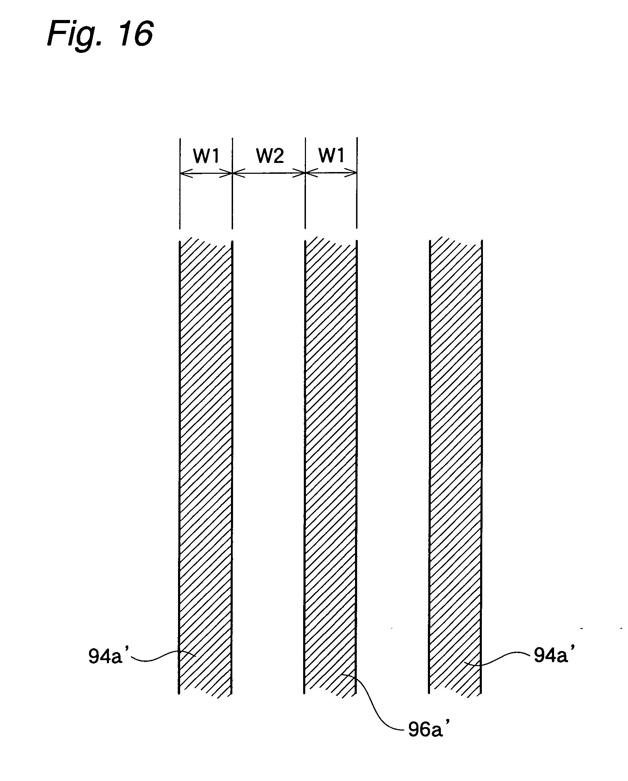

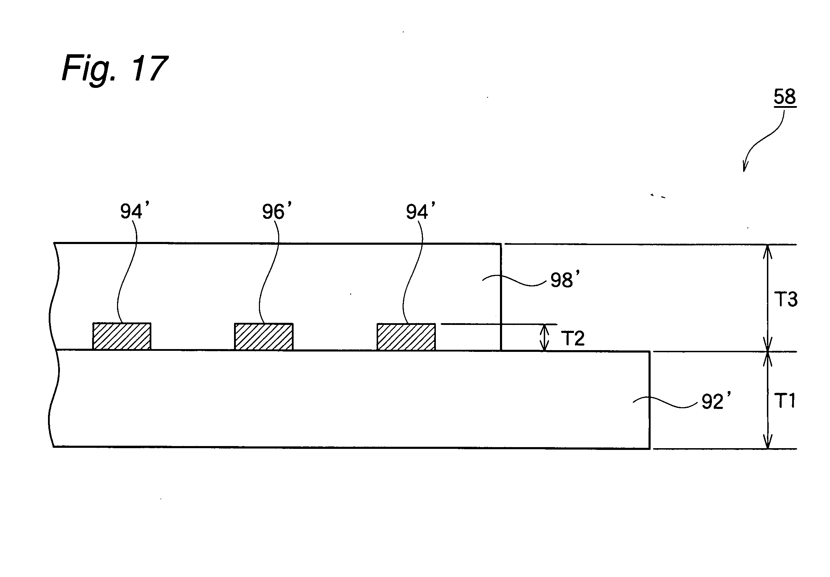

Fig. 14 is a schematic perspective view showing another example of the alcohol concentration detecting sensor 58 of the alcohol concentration detecting apparatus according to the present invention, Fig. 15 is a schematic top view showing an electrode wiring pattern in Fig. 14, Fig. 16 is an enlarged view showing a B portion in Fig. 15, and Fig. 17 is a partially enlarged sectional view taken along a C - C line in Fig. 14.

The alcohol concentration detecting sensor 58 according to the present example has basically the same structure as that of the alcohol concentration detecting sensor 58 according to the example shown in Figs. 9 to 12. Therefore, the same components are indicated as the reference numerals having a dash and detailed description thereof will be omitted.

The alcohol concentration detecting sensor 58 according to the present example comprises a substrate 92', electrode wiring patterns 94' and 96' formed on the substrate 92', and an insulating coat 98' covering the surfaces of the electrode wiring patterns 94' and 96'.

In this case, the electrode wiring patterns 94' and 96' are obtained by selectively etching a conductive metallic thin film formed on one of the surfaces of the substrate 92' through sputtering to provide a wiring pattern taking a predetermined shape.

While such a conductive metallic thin film is not particularly restricted, nickel, copper, platinum or the like can be used and the platinum is desirably used in consideration of an oxidation resistance or the like.

While a thickness T2 of each of the electrode wiring patterns 94' and 96' is not particularly restricted as shown in Fig. 17, moreover, in consideration of an efficiency in the formation of a thin film by the sputtering, it is preferably set to be 0.1 to 1.0 μm, and more preferably, 0.1 to 0.5 μm.

While the material of the substrate 92' is not particularly restricted, furthermore, in consideration of the fact that the material is not influenced by sputtering or the like, a glass substrate, a ceramics substrate such as alumina, a resin substrate or the like can be employed. While the thickness is not particularly restricted, it is preferably set to be 100 to 1000 μm, and more preferably, 250 to 600 μm in consideration of an insulating property, a strength or the like. In the present example, a thickness of 360μm was used. A size depends on the size of a sputtering device, and desirably, 2-inch and 4-inch square sizes can be used.

While the insulating coat 98' is not particularly restricted, moreover, it is preferably constituted by at least one minute insulating coat selected from $SiO_2$, $Al_2O_3$ and the like.

In this case, it is preferable that the insulating coat 98' should be formed by chemical vapor deposition (CVD).

By such a structure, it is possible to obtain, by the chemical vapor deposition (CVD), a very minute and thin insulating coat which is not influenced by a liquid to be inspected such as a gasoline or alcohol, for example, $SiO_2$, $Al_2O_3$ or the like, and the sensor itself can be thin, very small and compact.

While a thickness T3 of the insulating resin 98' is not particularly restricted as shown in Fig. 17, moreover, in consideration of the fact that the electrostatic capacitance of the insulating coat itself such as an insulating property, a strength and the like does not influence sensing, it is desirable that the thickness T3 should be smaller with an insulating property and a strength maintained. In the present example, T3 of 1 μm was employed.

By such a structure, it is possible to reduce the distance between the electrodes by using the electrode wiring patterns 94' and 96' formed on the substrate 92'. As is apparent from the Equation 2, therefore, the electrostatic capacitance $C_s$ can be increased and the excellent result of the measurement can be obtained.

In addition, the alcohol concentration detecting sensor is constituted by the substrate 92', the electrode wiring patterns 94' and 96' formed on the substrate 92', and the insulating coat 98' covering the surfaces of the electrode wiring patterns 94' and 96'. For this reason, the sensor itself is thin, very small and compact, and can be installed everywhere so that the degree of freedom of a design can be enhanced.

Since the surfaces of the electrode wiring patterns are covered with the insulating coat 98', furthermore, an insulation between the electrodes can be enhanced and is not influenced by a moisture, and shielding can be carried out so as not to be influenced by an electromagnetic wave generated from a body of a car or the like. Furthermore, the alcohol concentration can be measured accurately.

Moreover, the electrode does not directly come in contact with the liquid to be inspected such as a gasoline. Therefore, a defective operation can be prevented from being caused by a deterioration with passage of time, foreign matters in the gasoline or the like. Thus, it is possible to detect the alcohol concentration accurately and rapidly.

In addition, the substrate 92' is provided. Therefore, it is easy to assemble and attach the alcohol concentration detecting sensor into the apparatus.

Furthermore, it is possible to obtain an electrode wiring pattern having a thickness of 0.1 to 5μm by sputtering at a very small distance between the electrodes, for example, within a range of approximately 5μm to 50μm by the sputtering. Therefore, the electrostatic capacitance $C_s$ can be increased so that the excellent result of the measurement can be obtained.

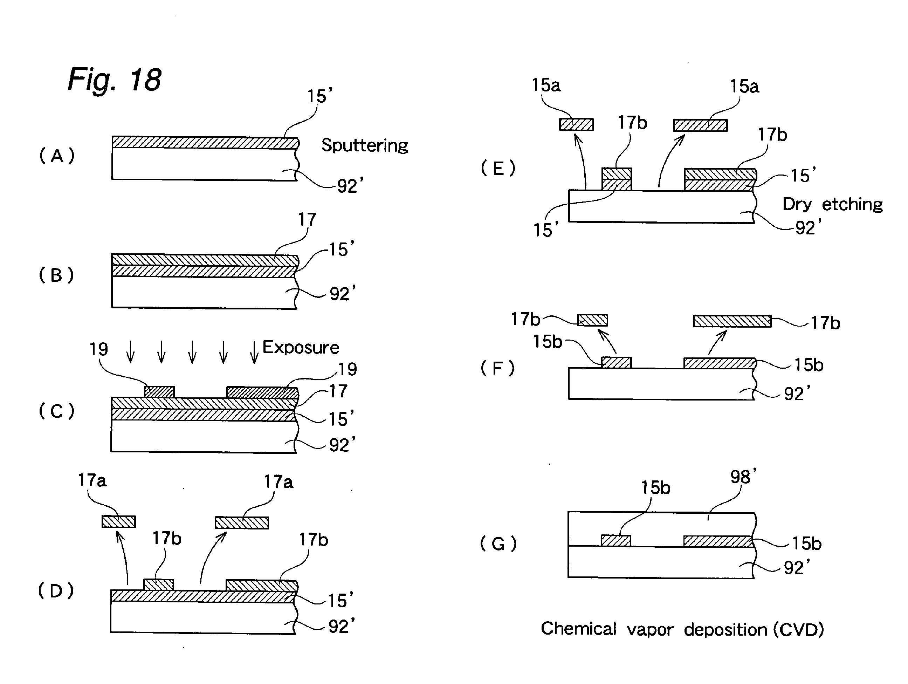

With reference to Fig. 18, description will be given to a method of manufacturing the alcohol concentration detecting sensor of the alcohol concentration detecting apparatus according to the present invention which has such a structure.

As shown in Fig. 18 (A), first of all, a conductive metallic thin film 15' is formed on one of the surfaces of the substrate 92' by the sputtering (a conductive metallic thin film forming step).

As shown in Fig. 18 (B), then, a photoresist 17 is applied onto the whole upper surface of the conductive metallic thin film 15' by using a spin coater (3000 rpm), for example (a photoresist applying step).

As shown in Fig. 18 (C), next, the photoresist 17 is exposed by ultraviolet rays to take a desirable electrode wiring pattern shape by using a photoresist mask 19 taking a shape corresponding to a predetermined wiring pattern, for example (a photoresist exposing step).

As shown in Fig. 18(D), then, a photoresist portion 17a thus exposed is dissolved and removed with a developing solution (a photoresist dissolving and removing step).

As shown in Fig. 18 (E), thereafter, a conductive metallic thin film portion 15a which is not covered with the photoresist 17b is subjected to a dry etching treatment by using an argon ion or the like, for example, and is thus removed to obtain a predetermined wiring pattern shape 15b (an etching step).

As shown in Fig. 18 (F), subsequently, the photoresist 17b is dissolved and removed with a dissolving and removing solution such as acetone (a photoresist dissolving and removing step).

As shown in Fig. 18 (G), finally, the insulating coat 98' is formed, by the chemical vapor deposition (CVD), on the surface from which the photoresist is removed (an insulating coat forming step).

By such a structure, it is possible to obtain an electrode wiring pattern having a thickness of 0.1 to 5 μm by the sputtering at a very small distance between the electrodes, for example, within a range of approximately 5 μm to 50 μm. Therefore, it is possible to easily supply, on a large scale, an alcohol concentration detecting sensor in which the electrostatic capacitance $C_s$ can be increased so that the excellent result of the measurement can be obtained. Furthermore, the sensor itself is thin, very small and compact, and can be installed everywhere so that the degree of freedom of a design can be increased.

While the preferred examples of the present invention have been described above, the present invention is not restricted thereto. For example, while the description has been given to the case in which the alcohol concentration in the gasoline is detected in the examples, the present invention can also be applied to the case in which the concentration of alcohol in another liquid to be inspected is to be detected. Thus, various changes can be made without departing from the scope of the present invention.

(Advantage of the Invention)

According to the present invention, it is possible to reduce a distance between the electrodes by using the electrode wiring pattern formed on the base material resin film. As is apparent from the Equation 2, therefore, the electrostatic capacitance $C_s$ can be increased so that the excellent result of the measurement can be obtained.

In addition, the alcohol concentration detecting sensor is constituted by the base material resin film, the electrode wiring pattern formed on the base material resin film, and the insulating resin covering the surface of the electrode wiring pattern. Therefore, the sensor itself is flexible, thin, very small and compact, and can be installed everywhere so that the degree of freedom of a design can be increased.

Furthermore, the surface of the electrode wiring pattern is covered with the insulating resin. Therefore, an insulation between the electrodes is excellent and is not influenced by a moisture, and shielding can be carried out in order to prevent the influence of an electromagnetic wave generated from the body of a car or the like, and furthermore, an accurate measurement for the alcohol concentration can be executed.

According to the present invention, moreover, it is possible to obtain an electrode wiring pattern having a very small distance between the electrodes, for example, within a range of approximately 5 μm to 50 μm by etching. Therefore, the electrostatic capacitance $C_s$ can be increased so that the excellent result of the measurement can be obtained.

According to the present invention, furthermore, it is possible to reduce a distance between the electrodes by using the electrode wiring pattern formed on the substrate. As is apparent from the Equation 2 described above, therefore, the electrostatic capacitance $C_s$ can be increased so that the excellent result of the measurement can be obtained.

In addition, the alcohol concentration detecting sensor is constituted by the substrate, the electrode wiring pattern formed on the substrate, and the insulating coat covering the surface of the electrode wiring pattern. Therefore, the sensor itself is thin, very small and compact, and can be installed everywhere so that the degree of freedom of a design can be increased.

Moreover, the surface of the electrode wiring pattern is covered with the insulating coat. Therefore, an insulation between the electrodes is excellent and is not influenced by a moisture, and shielding can be carried out in order to prevent the influence of an electromagnetic wave generated from the body of a car or the like. Furthermore, an accurate measurement for the alcohol concentration can be executed.

According to the present invention, furthermore, it is possible to obtain an electrode wiring pattern having a thickness of 0.1 to 5μm by sputtering at a very small distance between the electrodes, for example, within a range of approximately 5μm to 50μm by the sputtering. Therefore, the electrostatic capacitance $C_s$ can be increased so that the excellent result of the measurement can be obtained.

According to the present invention, moreover, it is possible to obtain, by chemical vapor deposition (CVD), a very minute and thin insulating coat which is not influenced by a liquid to be inspected such as a gasoline or alcohol, for example, $SiO_2$, $Al_2O_3$ or the like. Thus, the sensor itself can be thin, very small and compact.

Furthermore, the electrode does not directly come in contact with the liquid to be inspected such as a gasoline. Therefore, a defective operation can be prevented from being caused by a deterioration with the passage of time, foreign matters in the gasoline or the like. Thus, it is possible to detect the alcohol concentration accurately and rapidly.

In addition, the substrate is provided. Therefore, it is easy to assemble and attach the alcohol concentration detecting sensor into the apparatus.

According to the present invention, moreover, the positive and negative electrodes which are comb-toothed are formed to be alternately intricate. Therefore, the electrodes having a very small distance therebetween can be provided to be compact as a whole.

Accordingly, it is possible to obtain an electrode wiring pattern having a very small distance between the electrodes, for example, within a range of approximately 5μm to 50μm by sputtering, respectively. Therefore, the electrostatic capacitance $C_s$ can be increased so that the excellent result of the measurement can be obtained.

According to the present invention, furthermore, the alcohol concentration in the gasoline can be detected accurately and rapidly. Moreover, it is possible to control a torque to be constant by excessively adding a gasoline corresponding to the amount of addition of alcohol, for example, ethanol as an antiknocking agent. Thus, the present invention can produce many remarkable and peculiar functions and advantages, which is very excellent.

**Claims**

1. An alcohol concentration detecting apparatus in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency,
   wherein the alcohol concentration detecting sensor comprises an alcohol concentration detecting sensor body including a base material resin film, an electrode wiring pattern formed on the base material resin film, and an insulating resin covering a surface of the electrode wiring pattern.

2. The alcohol concentration detecting apparatus according to claim 1, wherein the alcohol concentration detecting sensor body is stuck onto a substrate.

3. The alcohol concentration detecting apparatus according to claim 1 or 2, wherein the electrode wiring pattern is obtained by selectively etching a conductive metallic foil laminated on one of surfaces of the base material resin film, thereby forming a wiring pattern taking a predetermines shape.

4. The alcohol concentration detecting apparatus according to any of claims 1 to 3, wherein the electrode wiring pattern has such a shape that positive and negative electrodes which are comb-toothed are alternately intricate.

5. An alcohol concentration detecting apparatus in which an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency,

wherein the alcohol concentration detecting sensor comprises a substrate, an electrode wiring pattern formed on the substrate, and an insulating coat covering a surface of the electrode wiring pattern.

6. The alcohol concentration detecting apparatus according to claim 5, wherein the electrode wiring pattern is obtained by selectively etching a conductive metallic thin film formed on one of surfaces of the substrate by sputtering, thereby forming a wiring pattern taking a predetermines shape.

7. The alcohol concentration detecting apparatus according to claim 5 or 6, wherein the insulating coat is formed by chemical vapor deposition (CVD).

8. The alcohol concentration detecting apparatus according to any of claims 5 to 7, wherein the electrode wiring pattern has such a shape that positive and negative electrodes which are comb-toothed are alternately intricate.

9. An alcohol concentration detecting method of detecting an alcohol concentration in a liquid to be inspected by using the alcohol concentration detecting apparatus according to any of claims 1 to 8,

wherein an alcohol concentration in the liquid to be inspected is detected by introducing a liquid to be inspected between electrodes of an alcohol concentration detecting sensor and by measuring a change in a specific inductive capacity of the liquid to be inspected between the electrodes with an oscillation frequency.

10. An alcohol concentration detecting method wherein the liquid to be inspected is a gasoline containing alcohol.

11. A method of manufacturing an alcohol concentration detecting sensor, comprising:

a conductive metallic foil sticking step of sticking a conductive metallic foil onto one of surfaces of a base material resin film;
a photoresist applying step of applying a photoresist onto a whole upper surface of the conductive metallic foil;
a photoresist exposing step of exposing the photoresist to take a desirable electrode wiring pattern shape by using a photoresist mask;
a photoresist dissolving and removing step of dissolving and removing the exposed photoresist portion with a developing solution;
an etching step of etching and removing a conductive metallic foil portion which is not covered with the photoresist, with an etchant;
a photoresist dissolving and removing step of dissolving and removing the photoresist; and
an insulating resin applying step of applying an insulating resin onto the surface from which the photoresist is removed, thereby obtaining an alcohol concentration detecting sensor body.

12. The method of manufacturing an alcohol concentration detecting sensor according to claim 11, further comprising a substrate sticking step of sticking the alcohol concentration detecting sensor body obtained at the insulating resin applying step, onto a substrate.

13. The method of manufacturing an alcohol concentration detecting sensor according to claim 11 or 12, wherein the base material resin film is a polyimide resin film.

14. The method of manufacturing an alcohol concentration detecting sensor according to any of claims 11 to 13, wherein the conductive metallic foil is a copper foil.

15. The method of manufacturing an alcohol concentration detecting sensor according to any of claims 11 to 14, wherein the insulating resin is constituted by at least one selected from an urethane resin, a polyimide resin and an epoxy type resin.

16. The method of manufacturing an alcohol concentration detecting sensor according to any of claims 11 to 15, wherein the electrode wiring pattern has such a shape that positive and negative electrodes which are comb-toothed are alternately intricate.

17. A method of manufacturing an alcohol concentration detecting sensor, comprising:

a conductive metallic thin film forming step of forming a conductive metallic thin film on one of surfaces of a substrate by sputtering;

a photoresist applying step of applying a photoresist onto a whole upper surface of the conductive metallic thin film;

a photoresist exposing step of exposing the photoresist to take a desirable electrode wiring pattern shape by using a photoresist mask;

a photoresist dissolving and removing step of dissolving and removing the exposed photoresist portion with a developing solution;

an etching step of dry etching and removing a conductive metallic thin film portion which is not covered with the photoresist;

a photoresist dissolving and removing step of dissolving and removing the photoresist; and

an insulating coat forming step of forming an insulating coat on a surface of the electrode wiring pattern from which the photoresist is removed, by chemical vapor deposition (CVD),

18. The method of manufacturing an alcohol concentration detecting sensor according to claim 17, wherein the substrate is constituted by at least one selected from ceramics, glass and a resin substrate.

19. The method of manufacturing an alcohol concentration detecting sensor according to claim 17 to 18, wherein the conductive metallic thin film is constituted by at least one selected from platinum, nickel, copper and titanium.

20. The method of manufacturing an alcohol concentration detecting sensor according to any of claims 17 to 19, wherein the insulating coat is constituted by at least one minute insulating coat selected from $SiO_2$, $Al_2O_3$ and the like.

21. The method of manufacturing an alcohol concentration detecting sensor according to any of claims 17 to 20, wherein the electrode wiring pattern has such a shape that positive and negative electrodes which are comb-toothed are alternately intricate.

Fig. 1

EP 1 548 426 A1

*Fig. 2*

EP 1 548 426 A1

Fig. 3

Fig. 4

*Fig. 5*

Ethanol vol %

A2 Heavy-duty gasoline

No7 Light-duty gasoline

Capacitance pF

Fig. 6

EP 1 548 426 A1

## Fig. 7

$$\frac{RA}{RA+2RB}$$

# Fig. 8

EP 1 548 426 A1

Fig. 9

58

98

92

11

96b

94b

13

C

C

*Fig. 10*

## Fig. 11

EP 1 548 426 A1

*Fig. 12*

58

94    96    94

98

T3

T2

92

T1

13

## Fig. 13

(A)

15
92

(B)

17
15
92

(C)

19 ↓ ↓ ↓ ↓ ↓ Exposure
19
17
15
92

(D)

17a        17a
17b        17b
15
92

(E)

15a        15a
17b        17b
15
15
Etching
92
15

(F)

17b        17b
15b        15b
92

(G)

15b    98
15b
92

(H)

15b    98
15b
92
13

EP 1 548 426 A1

EP 1 548 426 A1

Fig. 14

58

98'

96b'

94b'

C

92'

C

## Fig. 15

# Fig. 16

EP 1 548 426 A1

*Fig. 17*

## Fig. 18

(A)  Sputtering
15'
92'

(B)  17
15'
92'

(C)  19 ↓↓↓↓↓ Exposure
19
17
15'
92'

(D)  17a    17a
17b    17b
15'
92'

(E)  15a    17b    15a
17b
15'
15'    Dry etching
92'

(F)  17b    17b
15b    15b
92'

(G)  15b    98'
15b
92'

Chemical vapor deposition (CVD)

*Fig. 19*

*Fig. 20*

200

Printed circuit board

208 Insulating
material

Sensor case

O ring

204

Liquid to be
inspected

202

Temperature sensor

206

## INTERNATIONAL SEARCH REPORT

| | International application No. |
|---|---|
| | PCT/JP03/12504 |

**A. CLASSIFICATION OF SUBJECT MATTER**
Int.Cl$^7$ G01N27/22

According to International Patent Classification (IPC) or to both national classification and IPC

**B. FIELDS SEARCHED**

Minimum documentation searched (classification system followed by classification symbols)
Int.Cl$^7$ G01N27/00-27/24

Documentation searched other than minimum documentation to the extent that such documents are included in the fields searched
| | | | |
|---|---|---|---|
| Jitsuyo Shinan Koho | 1922-1996 | Toroku Jitsuyo Shinan Koho | 1994-2003 |
| Kokai Jitsuyo Shinan Koho | 1971-2003 | Jitsuyo Shinan Toroku Koho | 1996-2003 |

Electronic data base consulted during the international search (name of data base and, where practicable, search terms used)
JICST FILE (JOIS)

**C. DOCUMENTS CONSIDERED TO BE RELEVANT**

| Category* | Citation of document, with indication, where appropriate, of the relevant passages | Relevant to claim No. |
|---|---|---|
| X<br>Y | Norio MITSUMA et al., "Seiden Yoryoshiki Alcohol Nodo Sensor", Society of Automotive Engineers of Japan Gakujutsu Koenkai Mae Satsushu, 01 October, 1993 (01.10.93), No.936, pages 257 to 260 | 10<br>1-21 |
| X<br>Y | JP 4-350550 A (NGK Spark Plug Co., Ltd.), 04 December, 1992 (04.12.92), Full text; Figs. 1 to 7 (Family: none) | 10<br>1-21 |
| Y | WO 95/09361 A (Siemens AG.), 06 April, 1995 (06.04.95), Full text; Figs. 1, 4 & JP 9-503062 A Full text; Figs. 1, 4 | 3,4,6,8,<br>11-21 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br>17 October, 2003 (17.10.03) | Date of mailing of the international search report<br>28 October, 2003 (28.10.03) |
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

| | International application No. |
|---|---|
| | PCT/JP03/12504 |

C (Continuation).   DOCUMENTS CONSIDERED TO BE RELEVANT

| Category* | Citation of document, with indication, where appropriate, of the relevant passages | Relevant to claim No. |
|---|---|---|
| A | WO 99/57548 A  (Siemens AG.),<br>11 November, 1999 (11.11.99),<br>Full text; Fig. 1<br>& JP 2002-513930 A<br>Full text; Fig. 1 | 7,17-21 |
| A | US 5060619 A  (Osamu SAKURAI et al.),<br>29 October, 1991 (29.10.91),<br>Full text; Figs. 1 to 16<br>& JP 3-237353 A<br>Full text; Figs. 1 to 16 | 1-21 |
| A | US 5337018 A  (Frederich G. YAMAGISHI),<br>09 August, 1994 (09.08.94),<br>Full text; Figs. 1 to 2<br>& JP 6-265503 A<br>Full text; Figs. 1 to 2 | 1-21 |
| A | JP 62-182643 A  (Toyota Motor Corp.),<br>11 August, 1987 (11.08.87),<br>Full text; Figs. 1 to 4<br>(Family: none) | 1-21 |

Form PCT/ISA/210 (continuation of second sheet) (July 1998)